Europäisches Patentamt

European Patent Office

Office européen des brevets

(11) Veröffentlichungsnummer: **0 352 669**
**A2**

# (12) EUROPÄISCHE PATENTANMELDUNG

(21) Anmeldenummer: **89113490.0**

(22) Anmeldetag: **22.07.89**

(51) Int. Cl.4: **A61M 5/14**

(30) Priorität: **25.07.88 DE 3825201**

(43) Veröffentlichungstag der Anmeldung:
**31.01.90 Patentblatt 90/05**

(84) Benannte Vertragsstaaten:
**AT BE CH DE ES FR GB GR IT LI LU NL SE**

(71) Anmelder: **Sazunkewitzsch, Alexander, Dr.**
**Dreieichstrasse 39**
**D-6057 Dietzenbach(DE)**

(72) Erfinder: **Sazunkewitzsch, Alexander, Dr.**
**Dreieichstrasse 39**
**D-6057 Dietzenbach(DE)**

(74) Vertreter: **Linser, Heinz et al**
**Patentanwälte Heinz Linser Dipl. Ing.**
**Eckhardt Eyer Robert-Bosch-Strasse 12a**
**Postfach 10 22 10**
**D-6072 Dreieich(DE)**

## (54) Regulierbarer Schlauch-Klemmverschluss.

(57) Die Erfindung bezieht sich auf einen regulierbaren Schlauch-Klemmverschluß, insbesondere für Infusions- oder Transfusionsbestecke, bestehend aus einem einen Teil des Schlauches aufnehmenden Schlauchführungselement und einem darin zur Regulierung des Schlauchdurchflusses verschieblich gelagerten Klemmelement. Das Schlauchführungselement (4) weist eine über seine gesamte Länge sich erstreckende Öffnung (7) zur Einbringung eines Schlauchabschnittes (3) auf, welche seitlich durch eine U-förmige Nut begrenzt ist. Ein Schenkel der U-förmigen Nut ist mit einem Führungsteil (6) des Klemmelements (8) integriert, wobei das Klemmelement diesen Schenkel über einen Teil seiner Länge durchdringt und auf den während des Gebrauchs in der Nut liegenden Schlauch (3) klemmend einwirkbar ist.

Fig. 3

## Regulierbarer Schlauch-Klemmverschluß

Die Erfindung betrifft einen regulierbaren Schlauch-Klemmverschluß, insbesondere für Infusions- oder Transfusionsbestecke, bestehend aus einem einen Teil des Schlauches aufnehmenden Schlauchführungselement und einem darin zur Regulierung des Schlauchdurchflusses verschieblich gelagerten Klemmelement.

Regulierbare Schlauch-Klemmverschlüsse der eingangs genannten Art werden vorwiegend für Infusions- Transfusionsbestecke verwendet. Hierbei sind die Schläuche, mit denen die Strömungsverbindung zwischen der die Flüssigkeit enthaltende Flasche und dem Patienten hergestellt wird, durch den Schlauch-Klemmverschluß geführt und steril verpackt. Unmittelbar vor der Verwendung wird die Verpackung geöffnet, die Verbindung zur Flasche und zur in den Patienten eingeführten Nadel hergestellt. Über einen zwischengeschalteten Tropfbehälter erfolgt durch Abzählung der Tropfen die Regulierung des Schlauchdurchflusses, in dem das Klemmelement entsprechend von Hand auf dem Schlauchführungselement verschoben und verklemmt wird. Nach Entleerung der Flasche und Beendigung der Transfusion oder Infusion werden der Schlauch mit dem Schlauch-Klemmverschluß und der Flasche entsorgt, d.h. dieses bakteriell verseuchte Material wird in Spezialbehältern gesammelt und einer Vernichtungsanlage zugeführt und in den meisten Fällen verbrannt. Diese Materialien bestehen vorwiegend aus Kunststoff, so daß auch die Verbrennungsprodukte einer sorgfältigen Prüfung und Unschädlichmachung bedürfen.

Andererseits ist es nicht zulässig, den Schlauch aus dem Schlauch-Klemmverschluß zu ziehen und einen sterilen neuen Schlauch einzuführen, da bei diesem Vorgang die Sterilität des neuen Schlauches nicht gewahrt werden kann.

Der Schlauch-Klemmverschluß wird aus diesen Gründen nur einmal benutzt und sodann vernichtet.

In Fällen, in denen ein maximaler Flüssigkeitsstrom benötigt und beispielsweise eine Flasche mit einem Volumen von 50 ml verwendet wird, ist der Einsatz des Schlauch-Klemmverschlusses an sich nicht notwendig. Da sich dies jedoch nicht vorsehen läßt, befinden sich die Schlauch-Klemmverschlüsse mit dem zugehörigen Schlauch montiert in der sterilen Packung.

Transfusionen und Infusionen werden sehr häufig durchgeführt. So ist es beispielsweise nicht selten, daß ein Patient gleichzeitig mehrere Infusionen erhält, um die verschiedensten Körperfunktionen zu unterstützen oder zu beeinflussen.

Eine überschlägige Berechnung ergibt, daß allein in der Bundesrepublik Deutschland täglich mindestens ca. 1,5 Millionen Transfusionen und/oder Infusionen durchgeführt werden, wobei jeweils ein Schlauch-Klemmverschluss verwendet wird, welcher anschließend mit allen ungünstigen Begleiterscheinungen beseitigt werden muß. Hierzu zählen nicht nur der finanzielle Aufwand, sondern die Umweltbelastung, welche sich nicht ausschließen läßt.

Der Erfindung liegt die Aufgabe zugrunde, diesen schädlichen Kreislauf nahezu volständig zu beseitigen, in dem Mittel angegeben werden, derart, daß der Schlauch-Klemmverschluß vielfach verwendet werden kann, ohne daß die Sterilität der In- oder Transfuion negativ beeinflußt wird.

Die Lösung dieser Aufgabe besteht gemäß der Erfindung darin, daß bei dem eingangs genannten Schlauch-Klemmverschluß das Schlauchführungselement eine über seine gesamte Länge sich erstreckende Öffnung zur Einbringung eines Schlauchabschnittes aufweist.

Damit ist es möglich, den Schlauch-Klemmverschluß losgelöst von der sterilen Schlauchverpackung aufzubewahren und zu verwenden, da dieser mit keinem der Schlauchenden in Berührung kommt.

Ein Einfädeln des Schlauches durch den Klemmverschluß erübrigt sich; es wird vielmehr ein Teil oder ein kurzer Abschnitt des Schlauches in den Klemmverschluß gelegt und nach Beendigung des In- oder Transfusionsvorganges wieder entfernt, so daß der Schlauch-Klemmverschluß erneut verwendet werden kann.

Die Öffnung zur Einbringung eines Schlauchabschnittes ist vorteilhaft als eine sich über die gesamte Länge des Schlauch-Klemmverschlusses erstreckende U-förmige Nut ausgebildet. Der eingelegte Schlauch liegt daher auf einem ebenen Teil des Verschlusses, so daß sich eine günstige Fläche für die Klemmwirkung des Klemmelements ergibt.

In Weiterbildung der Erfindung ist ein Schenkel der U-förmigen Nut mit einem Führungsteil des Klemmelements integriert, wobei das Klemmelement diesen Schenkel über einen Teil seiner Länge durchdringt und auf den während des Gebrauchs in der Nut liegenden Schlauch klemmend einwirkt.

In einer besonderen Ausführungsform der Erfindung ist das Klemmelement als ein auf einer Achse gelagertes Rad ausgebildet, welches über den Umfang mit Rillen versehen sein kann, wobei das Führungsteil Führungsschienen oder Nuten zur Führung der Achse des Klemmelements aufweist. Die Führungsschienen oder Nuten zur Führung der Achse des Klemmelements, welche in der Ebene eines Schenkels der U-förmigen Nut liegen, verlaufen zu dem gegenüberliegenden Schenkel zur regulierbaren Klemmwirkung vorzugsweise linear keilförmig. Damit läßt sich die Klemmwirkung durch Veränderung der Position des Klemmelements kontinuierlich verstellen, so daß jeder Durchfluß entsprechend dem Schlauchquerschnitt einstellbar ist. Dies erfolgt in der Regel durch Zählung der Tropfen und dementsprechende Veränderung der Position des Klemmelements.

In einer weiteren Ausführungsform der Erfindung weisen die Führungsschienen oder Nuten zur Führung der Achse des Klemmelements in eine Ruhe- oder Null-Position an einem Ende eine Richtungsänderung auf, welche zu dem gegenüberliegenden Schenkel weggerichtet ist, wobei in den Schienen Rastelemente zur Fixierung der Achse des Klemmelements vorhanden sind. In eine solche Position, welche außerhalb der Klemmwirkung liegt, kann das Klemmelement gebracht werden, wenn seine Funktion nicht mehr benötigt wird, d.h. wenn der volle Schlauchquerschnitt für das Strömungsmittel benötigt wird.

Weiterhin ist es vorteilhaft, die U-förmige Nut oder ein Teil derselben verschließ- oder verriegelbar auszubilden. Hierdurch läßt sich einmal die Stabilität des U-förmigen Schenkels ausreichend sichern und zum anderen kann der Schlauch aus dem Schlauch-Klemmverschluß nicht unbeabsichtigt entfernt werden, wodurch die Funktion des Verschlusses und die exakte Justierbarkeit der Strömung sichergestellt ist.

Der Verschluß ist in einer Ausführungsform der Erfindung an einem Schenkel der U-förmigen Nut gelenkig verbunden und ist vorteilhaft in dem gegenüberliegenden Schenkel einrastbar, so daß dieser gleichzeitig als Versteifungselement des freien Schenkels ausgebildet ist.

Der Schlauch-Klemmverschluß nach der Erfindung besteht aus Kunststoff oder Metall, beispielsweise Edelstahl und kann vorteilhaft in Form eines Griffes ausgebildet sein, so daß das Klemmelement mit einer Hand einwandfrei justiert werden kann.

Die Nuten zur Führung der Achse des Klemmelements weisen Rasterungen zur Justierung des Klemmelements auf, so daß bestimmte und gemessene Positionen des Klemmelements fixiert werden können. Die Rasterungen sind dabei so ausgebildet, daß sie durch Verschieben des Klemmelements mit einem geringen Druck überwunden werden können.

Die Erfindung wird anhand der Zeichnung, in der ein Ausführungsbeispiel dargestellt ist, näher erläutert.

Hierbei zeigen:

FIGUR 1: eine perspektivische Darstellung des Schlauch-Klemmverschlusses;

FIGUR 2: einen Schnitt A-B nach FIGUR 1, und

FIGUR 3: ein weiteres Ausführungsbeispiel in der Darstellungsform nach FIGUR 1.

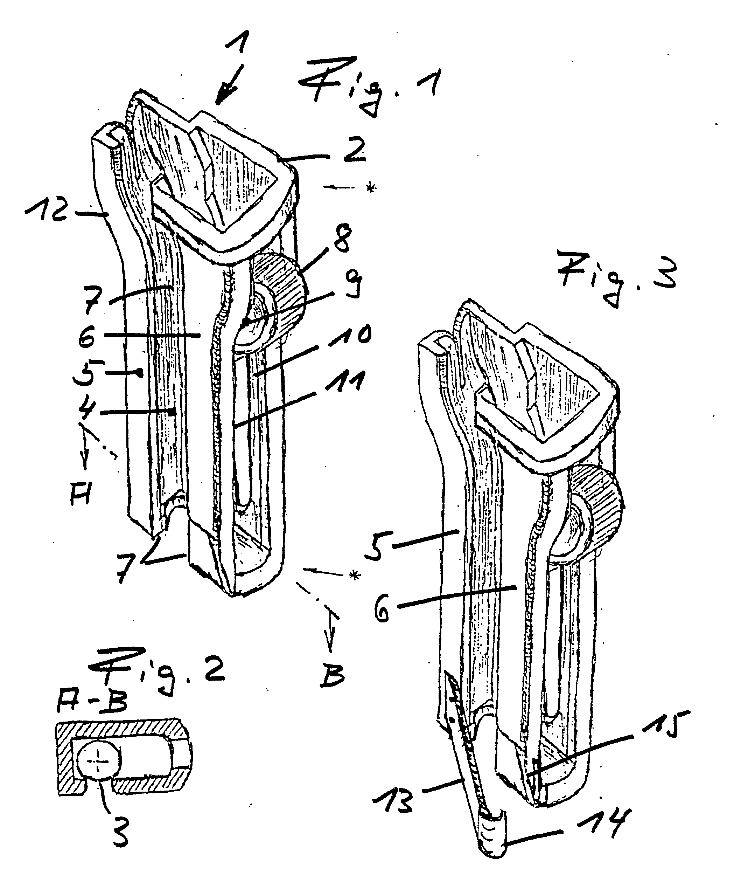

Der Schlauch-Klemmverschluß 1 besteht aus einem Gehäuse 2, welches aus Kunststoff oder Metall hergestellt sein kann. Wie aus Figur 2 ersichtlich ist, liegt im gebrauchsfertigen Zustand ein Schlauch 3 in einem Schlauchführungselement 4 mit den seitlichen Begrenzungen 5 und 6, welche als U-förmige Schenkel ausgebildet sind. Das Schlauchführungselement 4 ist mit einer Öffnung 7 versehen, welche sich über seine gesamte Länge erstreckt, so daß der Schlauchabschnitt 3, welcher ein Teil eines längeren Schlauches ist, bequem in diese Öffnung gelegt werden kann. Ein Klemmelement ist als ein Rad 8 dargestellt, dessen Achse 9 in den Nuten 10 und 11 geführt ist. Die Nuten 10 und 11 verlaufen zum Schlauchführungselement 4 keilförmig, so daß der zwischen der Oberfläche des Rades 8 und dem Schlauch 3 befindliche Abstand über die Länge des Schlauch-Klemmverschlusses linear von oben nach unten gemäß der Darstellung nach den Figuren 1 und 3, abnimmt.

Im oberen Teil des Schlauch-Klemmverschlusses 1, nämlich im gegenüberliegenden Teil der Auswölbung 12, ist eine nicht näher dargestellte Einrasterung für die Achse 8 vorgesehen, bei der das Rad 8 außer Eingriff zum Schlauch 3 in einer fixierten Ruhelage liegt.

Die Figur 3 zeigt die gleiche Ausführungsform nach Figur 1, jedoch mit einem Verschlußbügel 13, welcher an der seitlichen Begrenzung 5 schwenkbar befestigt ist und an seinem Ende eine hakenförmige Ausbildung 14 besitzt, die am Ende der seitlichen Begrenzung 6 bei 15 einrastet und die Öffnung 7 verschließt, wodurch gleichzeitig eine Verstärkungs- und Versteifungsbrücke zwischen den seitlichen Begrenzungen 5 und 6 gebildet wird.

Der Verschlußbügel 13 ist auf die dargestellte Ausführungsform nicht beschränkt.

Als ein besonders günstiges Material hat sich ein harter Kunststoff bewährt.

## Ansprüche

1. Regulierbarer Schlauch-Klemmverschluß, insbesondere für Infusions-oder Transfusionsbestecke, bestehend aus einem einen Teil des Schlauches aufnehmenden Schlauchführungselement und einem darin zur Regulierung des Schlauchdurchflusses verschieblich gelagerten Klemmelement, dadurch gekennzeichnet, daß das Schlauchführungselement (4) eine über seine gesamte Länge sich erstreckende Öffnung (7) zur Einbringung eines Schlauchabschnittes (3) aufweist.

2. Regulierbarer Schlauch-Klemmverschluß nach Anspruch 1, dadurch gekennzeichnet, daß die Öffnung (7) zur Einbringung eines Schlauchabschnittes (3) als eine sich über die gesamte Länge des Schlauch-Klemmverschlusses (1) erstreckende U-förmige Nut ausgebildet ist.

3. Regulierbarer Schlauch-Klemmverschluß nach Anspruch 1 oder 2, dadurch gekennzeichnet, daß ein Schenkel der U-förmigen Nut mit einem Führungsteil (6) des Klemmelements (8) integriert ist, wobei das Klemmelement diesen Schenkel über einen Teil seiner Länge durchdringt und auf den während des Gebrauchs in der Nut liegenden Schlauch (3) klemmend einwirkbar ist.

4. Regulierbarer Schlauch-Klemmverschluß nach Anspruch 1, 2 oder 3, dadurch gekennzeichnet, daß das Klemmelement als ein auf einer Achse gelagertes Rad ausgebildet ist, wobei das Führungsteil Führungsschienen oder Nuten (10, 11) zur Führung der Achse des Klemmelements (8) aufweist.

5. Regulierbarer Schlauch-Klemmverschluß nach Anspruch 1 oder einem der voranstehenden Ansprüche, dadurch gekennzeichnet, daß die Führungsschienen oder Nuten (10, 11) zur Führung der Achse (9) des Klemmelements 8, welche in der Ebene eines Schenkels der U-förmigen Nut liegen, zu dem gegenüberliegenden Schenkel zur regulierbaren Klemmwirkung keilförmig verlaufen.

6. Regulierbarer Schlauch-Klemmverschluß nach Anspruch 1 oder einem der voranstehenden Ansprüche, dadurch gekennzeichnet, daß die Führungsschienen oder Nuten (10, 11) zur Führung der Achse (9) des Klemmelements in eine Ruhe- oder Null-Position an einem Ende eine Richtungsänderung aufweisen, welche zu dem gegenüberliegenden Schenkel weggerichtet ist, wobei in den Schienen Rastelemente zur Fixierung der Achse des Klemmelements (8) vorhanden sind.

7. Regulierbarer Schlauch-Klemmverschluß nach Anspruch 1 oder einem der voranstehenden Ansprüche, dadurch gekennzeichnet, daß die U-förmige Nut oder ein Teil derselben verschließbar ausgebildet ist.

8. Regulierbarer Schlauch-Klemmverschluß nach Anspruch 1 oder einem der voranstehenden Ansprüche, dadurch gekennzeichnet, daß der Verschluß (13) an einem Schenkel der U-förmigen Nut gelenkig verbunden ist.

9. Regulierbarer Schlauch-Klemmverschluß nach Anspruch 1 oder einem der voranstehenden Ansprüche, dadurch gekennzeichnet, daß der an einem Schenkel der U-förmigen Nut gelenkig verbundene Verschluß (13) in dem gegenüberliegenden Schenkel einrastbar ist und gleichzeitig als Versteifungselement des freien Schenkels ausgebildet ist.

10. Regulierbarer Schlauch-Klemmverschluß nach Anspruch 1 oder einem der voranstehenden Ansprüche, dadurch gekennzeichnet, daß dieser aus Kunststoff oder Metall, insbesondere Edelstahl besteht und als Handgriff gestaltet ist.

11. Regulierbarer Schlauch-Klemmverschluß nach Anspruch 1 oder einem der voranstehenden Ansprüche, dadurch gekennzeichnet, daß die Nuten zur Führung der Achse des Klemmelements Rasterungen zur Justierung des Klemmelements aufweisen.

Fig. 1

Fig. 3

Fig. 2

A - B